# EUROPEAN PATENT APPLICATION

(11) **EP 3 673 790 A1**
(43) Date of publication of application: **01.07.2020**
(21) Application number: 18215826.1
(22) Date of filing: 24.12.2018
(51) Int. Cl.: A61B 5/00, A45D 44/00, A61B 90/00

(54) **DEVICE TO DETECT AND MARK SKIN AREAS OF INTEREST FOR USER SELF-DETECTION OR OTHER DEVICES**

(71) Applicant: Koninklijke Philips N.V., 5656 AG Eindhoven (NL)
(72) Inventor: JURNA, Martin, 5656 AE Eindhoven (NL); BOURQUIN, Yannyk Parulian Julian, 5656 AE Eindhoven (NL)
(74) Representative: de Haan, Poul Erik

(57) **Abstract**

The invention provides a screening device (100) for screening a skin part, wherein the screening device (100) comprises a first sensor (110), a first control system (120), and a marking device (130), wherein:
- the first sensor (110) and first control system (120) are configured to determine a skin parameter of a skin part when the first sensor (110) is configured at a predetermined distance (d1) from the skin, wherein the skin parameter is a skin condition parameter of the skin part, and wherein the skin parameter is selected out of a predetermined set of skin parameters; and
- the marking device (130) is configured to generate to the skin part a first marking, selected out of a set of first markings, according to a predetermined relation between skin parameters and first markings.

## Description

### FIELD OF THE INVENTION

The invention relates to a screening device and to a kit of parts comprising such screening device. The invention further refers to a system comprising such screening device. Yet further, the invention relates to a method for screening, as well as to a computer program product that can execute or have executed such method.

### BACKGROUND OF THE INVENTION

On the skin, especially facial skin, there are multiple areas that are easily visually (or by touch) recognized by the consumer (e.g. pigmentation, pimples, wounds, scars, etc.). Nevertheless, there are also areas that are not/less easily recognizable (e.g. early pimples, low and high blood perfused areas, fragile or sensitive skin, etc.). Especially these areas are particularly of interest to detect and monitor closely. Having knowledge about the location of these areas and having the possibility of finding back the location provides the user or a treatment device with the possibility to treat certain specific areas before e.g. they become noticeable, or preventing the area to develop into a less favorable stage. There are technologies available to detect certain areas of interest. However, these detectors often are not or cannot be integrated within a treatment device, due to their complexity and bulkiness, and/or are unable to communicate the location on a clear and simple way to the user.

Systems for marking the surface of a patient's tissue are known in the art. WO2018/055401, for instance, describes a marking apparatus for marking tissue imaged through an imaging device, including: a viewing element including an imaging window providing an imaging zone through which tissue can be imaged; a marker implement; a coupling device to which the marker implement is connected; the coupling device being configurable to position the marker implement in a first position substantially out of the imaging window and a second position in the imaging zone of the imaging window; the marker implement being able to apply a mark when in the second position and thereby to mark the tissue in the imaging zone while the apparatus remains in position on the tissue. The apparatus provides a mechanism by which it is possible to apply a mark on the surface of imaged tissue (such as skin) while keeping the imaging device in position on the tissue, thereby enabling the mark to be applied under image-guidance.

### SUMMARY OF THE INVENTION

Hence, it is an aspect of the invention to provide an alternative screening device and/or system, which preferably further at least partly obviate one or more of above-described drawbacks. Likewise, it is an aspect of the invention to provide an alternative screening method, which preferably further at least partly obviates one or more of above-described drawbacks. The present invention may have as object to overcome or ameliorate at least one of the disadvantages of the prior art, or to provide a useful alternative.

Amongst others, herein a skin analysis (device) is proposed with integrated skin marking device, for instance to help the user to understand its skin and where the skin markings can assist other personal care devices or (in-depth) analysis devices without having the need of sophisticated communication between devices.

Hence, in a first aspect the invention provides a device ("screening device" or "first device") for screening a skin part, wherein the device comprises a first sensor, a first control system, and a marking device, wherein:
- the first sensor and first control system are configured to determine a skin parameter of a skin part (when the first sensor is configured at a predetermined distance (d1) from the skin), wherein especially the skin parameter is a skin condition parameter of the skin part, and wherein the skin parameter is selected out of a predetermined set of skin parameters; and
- the marking device is configured to generate to the skin part a first marking ("marking"), selected out of a set of first markings, especially according to a predetermined relation between skin parameters and first markings.

With such device, it is possible to provide a marking which provides information about the condition of the (sensed) skin part. Hence, the user may derive from the marker, either directly, or with the aid of another device, information about the condition of the (sensed) skin part. With the sensor, the device may determine e.g. whether the part of a skin is healthy or not. On the basis thereon, the user may take measure or omit actions. Alternatively or additionally, on the basis thereon a second device may execute a more thorough or more dedicated screening. Alternatively or additionally, on the basis thereon a second device may execute a specific action.

The device is especially a "screening device" or "skin screening device". Instead of the term "device" also the term "apparatus" may be used. The device will further essentially be indicated as "screening device".

Especially, the device is configured for screening a skin part. The term "skin" especially refers to an organ of the integumentary system, especially of a mammal, even more especially of a human, made up of multiple layers of ectodermal tissue, and guards the underlying muscles, bones, ligaments and internal organs. Especially, the skin may comprise two primary layers: the epidermis, which provides waterproofing and serves as a barrier to infection; and the dermis, which serves as a location for the appendages of skin. However, also the hypodermis may be considered part of the skin. In arthropods, the hypodermis is an epidermal layer of cells that secretes the chitinous cuticle.

For instance, the term skin may refer to facial skin, skin of the abdomen, skin of a limb, such as an arm or a leg, etc.. The device may screen several skin parts. In general, the term "skin part" may refer to part of skin of e.g. the face, of the abdomen, of a limb, etc.. The skin part that may be sensed with the sensor may in embodiments be in the order of 0.25-100 cm², such as in the range of 0.25-4 cm². Of course, when moving the device over the skin, the device may screen adjacent screen parts. In this way, the device may be able to screen parts of larger areas than indicate above. However, the condition of the skin may differ over an abdomen, over the face, over a limb, etc. Hence, with the device it may be possible to indicate the condition of skin parts in the order 0.25-100 cm², such as in the range of 0.25-4 cm² individually. Hence, each (individual) skin part may be assigned a related condition (see further also below).

Especially, the screening device is be configured to be in contact with the skin during screening. Hence, during use of the screening device, at least part of the screening device may be in physical contact with the skin. In this way, the screening device may be configured over a skin part. In this way, the screening device may also determine the skin parameter of the skin part over which the screening device is configured. In embodiments, see also below, in the same configuration over the skin part wherein the screening device determines the skin parameter, the screening device may also provide the first marking.

As indicated above, the screening device comprises a first sensor, a first control system, and a marking device.

The first sensor may especially be configured to sense the skin part (over which the (screening device with the) first sensor is configured. The first sensor may generate a first sensor signal, from which the first parameter may be derived (by the control system). The first sensor may be configured to sense continuously or to sense intermittently (see also below). In embodiments, the first sensor may be configured to sense in response to an instruction via a user interface. In embodiments, the first sensor maybe configured to sense in response to a sensor signal of a movement sensor. The control system may control the first sensor, e.g. to have the sensor sense during a predetermined sensing period, such as e.g. 0.1-10 seconds, like 0.1-5 seconds, such as e.g. 0.2-2.5 seconds. As will be clear from the below, the term "sensing" may in embodiments comprise a signal generation action and signal receiving action. The signal receiving action may in embodiments overlap in time with at least part of the time the signal generation action is executed. Based on the first sensor signal generated during the sensing period, the control system may determine the skin parameter.

In embodiments, the first sensor may comprises one or more of a camera, a thermal imager, a topographical imager, an ultrasound sensor, a radiofrequency sensor, a capacitance sensor, etc.

The first sensor may include a generating element and a receiving element. For instance, the generating element may comprise one or more light sources, such as solid state light sources, an optionally optics, configured to generate one or more of ultraviolet (UV) radiation, visible radiation, and infrared (IR) radiation. The receiving element may be an optical sensor configured to sense one or more of reflected light, polarization of reflected light, luminescence, scattering of the light, etc. etc.. The generating element may be configured to send a signal, such as radiation and/or sound and/or vibration to the skin part. The receiving element may be configured to detect a change to the signal due to interaction with the skin (like reflection or light, changing of polarization, etc.). Alternatively or additionally, the receiving element may be configured to detect a signal from the skin due to interaction of the signal of the generating element with the skin (e.g. luminescence).

The generating element and a receiving element may be integrated in a single device. However, in embodiments the first element may also comprise a generating element and a receiving element that are different devices, which may be configured at different positions within the screening device.

The first sensor may be controlled by the control system (see also below). The control system may thus in embodiments be configured to control the generating element and the receiving element. The receiving element may be configured to generate a first sensor signal.

The term "first sensor" may also refer to a plurality of (different) first sensors, which may embodiments e.g. be spatially configured around the marking device. In embodiments, a plurality of (different) first sensors may be configured (in a regular configuration) around the marking device.

The term "generating element" may also refer to a plurality of (different) generating elements, which may embodiments e.g. be spatially configured around a receiving element. In embodiments, a plurality of (different) generating elements may be configured (in a regular configuration) around the receiving element. In embodiments, a plurality of (different) generating elements may be configured (in a regular configuration) around the marking device.

The term "receiving element" may also refer to a plurality of (different) receiving elements, which may in embodiments be spatially configured around a generating element. In embodiments, a plurality of (different) receiving elements may be configured (in a regular configuration) around the generating element. In embodiments, a plurality of (different) generating elements may be configured (in a regular configuration) around the marking device.

The first sensor, or at least part thereof, may be in physical contact with the skin or may be configured at a distance of the skin (when the screening device is configured on the skin). Especially, the screening device may be configured to be in contact with the skin during screening. The screening device may include one or more distance holders, e.g. to keep the sensor or at least part thereof, at a non-zero distance from the skin and/or to keep the marking device, or at least part thereof, at a non-zero distance from the skin. For instance, the screening device may include a housing with an opening, which when the housing is in contact with the skin in a functional configuration, the first sensor is in physical contact with the skin or is not in physical contact with the skin. However, the screening device may be configured such, that during operation of the device, such as with part of the device in physical contact with the skin (in a functional configuration), there is essentially a fixed distance of the first sensor to the skin. This distance may be zero or non-zero. This distance is herein also indicated as predetermined distance. Especially, in embodiments the predetermined distance (d1) is selected from the range of 0-50 mm, such as selected from the range of 1-25 mm. In embodiments wherein the sensor comprises a generating element and a receiving element, the respective distances to the skin part may be selected from this predetermined distance ranges of 0-50 mm, such as selected from the range of 1-25 mm. The respective distances (of the generating element and a receiving element) may in embodiments be the same, but may also differ in other embodiments. Hence, during use the screening device may be configured on the skin, but the first sensor (or in embodiments the generating element and a receiving element of the first sensor) may be configured at a non-zero distance from the skin (part), such as at about 1 mm (or more) from the skin (part).

The distance holder(s) may be an edge, with the sensor and marking device configured within a cavity which may be at least partly be defined by the edge. When the screening device is configured on the skin, the cavity may be closed by the skin. In this way, external influences, such as ambient light, may be minimized. Hence, the screening device may be configured to be used on the skin. Therefore, for screening, the screening device may be configured on the skin, to determine the skin parameter of the skin over which the screening device is configured.

The screening device may also comprise a control system (see also above). Likewise, the second device (see below) and/or the system (see below) may comprise a control system.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The system, or apparatus, or device (screening device or second device) may execute an action in a "mode" or "operation mode" or "mode of operation". Likewise, in a method an action or stage, or step may be executed in a "mode" or "operation mode" or "mode of operation". The term "mode" may also be indicated as "controlling mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

The control system may include a memory, a processing device (or "processor" or "processor system" or "controller" or "control system"), and a user interface, and optionally a display. Examples of user interface devices include a manually actuated button, a display, a touch screen, a keypad, a voice activated input device, an audio output, an indicator (e.g., lights), a switch, a knob, a modem, and a networking card, among others. Especially, the user interface device may be configured to allow a user instruct the device or apparatus with which the user interface is functionally coupled by with the user interface is functionally comprised. The user interface may especially include a manually actuated button, a touch screen, a keypad, a voice activated input device, a switch, a knob, etc., and/or optionally a modem, and a networking card, etc. The user interface may comprise a graphical user interface. The term "user interface" may also refer to a remote user interface, such as a remote control. A remote control may be a separate dedicate device. However, a remote control may also be a device with an App configured to (at least) control the system or device or apparatus.

The controller/processor and the memory may be any type. The processor may be capable of performing the various described operations and executing instructions stored in the memory. The processor may be an application-specific or general-use integrated circuit(s). Further, the processor may be a dedicated processor for performing in accordance with the present system or may be a general-purpose processor wherein only one of many functions operates for performing in accordance with the present system. The processor may operate utilizing a program portion, multiple program segments, or may be a hardware device utilizing a dedicated or multi- purpose integrated circuit.

Especially, the control system may be configured to control the first sensor and the marking device.

The control system of the screening device may especially be configured to determine on the basis of a first sensor signal of the first sensor a skin parameter. The skin parameter may be indicative of a condition of the skin part.

Hence, in embodiments the first sensor and first control system are especially configured to determine a skin parameter of a skin part when the first sensor is configured at the predetermined distance (d1) from the skin (see also above).

Especially, the skin parameter is a skin condition parameter of the skin part. In embodiments, the skin parameter may be selected from the group consisting of dermatological diseases of the skin and physical conditions of the skin. This skin condition is not necessarily negative. The absence of certain aspects or the compliance with certain predetermined values may also lead to a condition like "healthy", "strong", "flexible", etc.. The skin parameter may in embodiments thus also contain such information.

Further, as will be clear to a person skilled in the art, a skin part may have several conditions at the same time or a condition may reflect several aspects. Hence, the term "skin parameter" may also refer to a plurality of different skin parameters. Dependent upon the type of sensor, one or more, especially two or more, different skin parameters may be determined by the screening device. Concomitant with the skin parameter may be an evaluation. Hence, the skin parameter may (also) include an evaluation, like large/average/small, or a binary evaluation, like absent/present, or normal/abnormal, etc., or a numerical evaluation, like a scaling, like in the range of x-y, such as 1-5, etc. etc. In specific embodiments, the skin parameter may be selected from local pigmentation excess, a local pigmentation scarcity, a local pigmentation anomaly, pimple formation, a pimple, a wound, a scar, a local low blood perfused area, a local high blood perfused area, a local blood perfusion anomaly, a fragile skin part, a sensitive skin part, wrinkles, skin hydration, skin oiliness, and a local skin elasticity anomaly. Note that such skin parameters may thus be multi-dimensional, such as "local pigmentation anomaly"/"no local pigmentation anomaly", or "local pigmentation anomaly severity 2"/"local pigmentation anomaly severity 1"/"local pigmentation anomaly severity" (i.e. "no local pigmentation anomaly"), etc. etc. Herein, local pigmentation excess or scarcity may indicate a relatively increased or relatively decreased local pigmentation, relative to an average, whereas a local pigmentation anomaly may e.g. be a disease. Hence, the former terms may basically refer to a local pigmentation variation. Instead of the term "disease" also the term "illness" may be applied.

In further specific embodiments, the skin parameter may be selected from the group consisting of skin gloss, skin oiliness, and skin hydration, such as especially skin oiliness. Further, in embodiments with the device (sensor) it may be possible to quantitatively estimate skin gloss. The term "skin gloss" herein refers to gloss of the skin but may also refer to "skin oiliness". Hence, the term "skin gloss" herein may also be defined as "skin parameter especially selected from one or more of the group consisting of skin gloss and skin oiliness". The values that may be measured with the device as described herein may reflect skin gloss and skin oiliness, as skin gloss may be related to skin oiliness. Herein, the term "skin gloss" is sometimes used to indicate both skin gloss or skin oiliness. Hence, in embodiments the term skin gloss may refer to skin gloss or skin oiliness, or especially to skin gloss. Hence, especially the skin parameter may be selected out of a predetermined set of skin parameters.

The screening device may also comprise a marking device. Especially, the marking device may be configured to generate to the skin part a first marking. In general, the marking device may be configured to generate to the skin part the first marking when the device is configured in physical contact with the skin. This first marking may be (provided) on the skin, and/or may be in the skin, and/or may be under the skin. For instance, one or more of a dye and a pigment may be applied to the skin. Alternatively or additionally, some minor local damages may be provided to the skin. Alternatively or additionally, it may also be possible to inject material into the skin, or even below the skin, which may e.g. be biodegradable.

The first marking is such, that device, may - based on the marking - determine the condition of the skin part. In embodiments, the first marking may have a specific color which is provided according to a predetermined relation between skin parameters and first markings. Alternatively or additionally, marking may have a specific pattern which is provided according to a predetermined relation between skin parameters and first markings.

The first marking may thus have a shape and color. As different shapes may be applied and as also different colors or shading may be applied, different types of conditions and/or different severity of a condition or of conditions may be indicated with the first marking. Alternatively or additionally, the first marking may include a kind of matrix (2D) code, such as a QR code, a ShotCode, as Dotcode, or other type of code. Hence, in embodiments the first marking may include a kind of bar code or QR code or other type of code. Further, the term "first marking" or the term "marking" or the term "second marking" (see also below), may in embodiments refer to a plurality of (different) first markings, (different) markings, or (different) second markings, respectively. Hence, the first marking is selected out of a (predetermined) set of first markings, according to a predetermined relation between skin parameters and first markings. In this way, the second device (see also below) can derive from the first marking the condition of the skin and e.g. execute a dedicated skin treatment of the skin part. The treatment may differ for different skin parts

In specific embodiments, the first marking may (additionally) contain information related to one or more of a depth of the condition, an extent over the skin of the condition, a severity of the condition, a stage of the condition, etc.. The depth of the condition may e.g. relate to the depth relative to the stratum corneum. The extent of the condition may e.g. refer to the area, especially in the plane of the stratum corneum, of the skin part for which the first marking provides information. The extent of the condition may also refer to the shape of the condition, the orientation of the direction. The extent of the condition may in embodiments also include the location of the condition

The extent may be physically indicated, such as by markings indicating the area for which the condition has been sensed. The extent may also be indicated by providing information in the marking, such as indicated above with color, shape, and (bar or QR) coded information, etc.. The severity has also been indicated above with examples.

Alternatively or additionally, the first marking may contain information about the conditions of the treatment to be executed, such as one or more of intensity of the treatment, the length in time of a treatment, the number of treatments (including optionally the associated time of each treatment). Hence, in embodiments the first marking may contain information about a treatment scheme.

The phrase "the first marking contains information" and similar phrases may especially indicated that the first marking is configured such, like in terms of color, geometry, etc.., that the second device can derive information therefrom based on the predetermined relation between the skin parameters and the first markings.

Above, the option of using colors to indicate type of dermatological disease (or absence) thereof and/or severity, and/or other aspects such as depth or extent, of such disease has been mentioned. Hence, in specific embodiments the marking device is configured to generate to the skin part a colored first marking, selected out of a (predetermined) set of colored first markings, according to a predetermined relation between skin parameters and the colored first markings. Note that the term "colored first marking" may also refer to a first marking having parts with different colors.

Herein the term "color" may refer to any known color, including black and white. For instance, a black pigment may be used. However, also a white pigment may be used, or a colored (non-white or non-black) pigment may be used. Alternatively or additionally, a luminescent material may be used, which may e.g. luminesce upon irradiation with blue light or UV radiation.

Hence, in embodiments the screening device comprises a plurality of cartridges configured to host one or more of pigments and luminescent materials.

Above, also the option of using patterns, such as a coded pattern, to indicate type of dermatological disease (or absence) thereof and/or severity, and/or other aspects such as depth or extent of such disease has been mentioned. Hence, in specific embodiments the marking device is configured to generate to the skin part a patterned first marking, selected out of a (predetermined) set of patterned first markings, according to a predetermined relation between skin parameters and the patterned first markings.

Especially, as indicated above the first markings will be provided in dependence of the skin parameter.

In specific embodiments, the screening device may thus comprise a printer, wherein the printer is configured to print patterned first markings, or colored first markings, or both patterned and colored first markings, according to a predetermined relation between skin parameters and the patterned first markings. Alternatively or additionally, the screening device may comprise a spray device, wherein the spray device is configured to spray patterned first markings, or colored first markings, or both patterned and colored first markings, according to a predetermined relation between skin parameters and the patterned first markings. Hence, in embodiments the marking device may comprise one or more of a printer and a spray device.

However, the screening device may be configured such, that during operation of the device, such as with part of the device in physical contact with the skin (in a functional configuration), there is essentially a fixed distance of the marking device to the skin. This distance may be zero or non-zero. This distance is herein also indicated as second predetermined distance (d2). Especially, in embodiments the second predetermined distance (d2) is selected from the range of 0-50 mm, such as selected from the range of 1-25 mm. Hence, during use the screening device may be configured on the skin, but the marking device may be configured at a non-zero distance from the skin (part), such as at about 1 mm (or more) from the skin (part). However, the marking device may also comprise a movable element, for instance an element that is configured at the second predetermined distance, but temporary contacts the skin (part) to provide the first marking. For instance, the marking device may comprise a kind of (digital) stamping device. As indicated herein, the marking device is especially controlled by the control system.

The first marking can be used to indicate also the extent of the skin part associated with the skin parameter. For instance, a skin part with local blood perfusion anomaly may be indicated as such by a color and/or pattern and/or coded pattern. The extent of that skin part may also be indicated with a color and/or pattern and/or (the same) coded pattern. For instance, the second device can according to the predetermined relation conclude that the extent is 4 cm², with the respective first marking in the middle of the square of 4 cm². Alternatively (or additionally), however, an additional marking may be used to indicate the extent. This second marking may e.g. include a closed line surrounding the relevant skin part, or may e.g. include a plurality of markings surrounding the relevant skin part, etc.. Hence, the second marking is herein also indicated as "area marking". Therefore, in embodiments the marking device may be configured to generate to the skin part an area marking for indicating the area of the skin part associated to the (defined) skin parameter.

Embodiments above in relation to the first marking, such as in relation to color, shape, pattern, etc., may also apply to the area marking.

In specific embodiments, the screening device may be configured to generate the marking related to a specific skin part while (or (just) after) determining the skin part. Here, "marking" may refer to the first marking and the optional area marking.

Further, the control system may especially be configured to control the marking device in dependence of the skin parameter.

In embodiments, the screening device may be designed to screen in a kind of batch process. For instance, the screening device should be kept essentially on or over a part of the skin, to determine the skin parameter and to provide the first marking, before the screening device may be oved to another position on or over part of the skin. To this end the screening device may also include a light source and/or sound source and/or vibration source, to provide to the user a light signal and/or sound signal and/or vibration signal (by vibrating at least part of the screening device), to inform the user that determination and concomitant providing of the first marking has been executed (and the screening device may be moved to another position).

In other embodiments, the screening device may be designed to screen in a kind of continuous process. For instance, the screening device may (slowly) be moved (by hand) over the skin while allowing screening and providing the first marker(s). To this end the screening device may comprise rolling elements, and optionally a motor for controlling the rotational speed of the rolling elements. In such way, the speed over the skin may be controlled and/or the position of the first marking (and option further markings) may be controlled as function of the rotational speed.

In embodiments, the first sensor has a field of view (FV), wherein the marking device is configured to provide (to the skin part) the first marking and/or the area marking (as also defined herein) within the field of view (FV), or wherein the marking device is configured to provide (to the skin part) a plurality of the first marking and/or a plurality of the area markings at an edge of the field of view (FV) or outside the field of view (FV). For instance, the marking device may comprise multiple nozzles for printing (or spraying) to provide a marking that is configured defining or surrounding the skin part. Note that the term "surrounding" does not necessarily mean a closed feature, but may also include a plurality of features that are configured as different positions in between which the skin part may be situated (such as e.g. four markings or four parts of a marking defining a rectangular skin part).

Hence, the screening device may especially be configured such that when the marking is provided, a second device knows where the skin part is and/or what the associated skin parameter are; see further also below.

As indicated above, the screening device may especially be used as first device preceding a later action with a second device. The second device may be a specific analysis device and/or s specific treatment device. Hence, a first action, preceding the second action of the second device, may be the generation of the first marking to the skin (part) (based on the screening with the first sensor).

Note that herein the term "treatment" and similar terms may in embodiments refer to non-medical treatments.

Hence, in yet a further aspect, the invention also provides a kit of parts comprising the screening device as defined herein and the second device. Especially, in embodiments the second device is configured to execute a second action selected from the group consisting of executing an analysis and executing a treatment, wherein the second action is selected out of a predetermined set of second actions, wherein the second device comprises a second sensor configured to generate a second sensor signal in response to one or more of (a) the first marking, (b) the background of the first marking, and (c) the first marking and its background, wherein the second device further comprises a second control system configured to select the second action in response to the second sensor signal based on a predefined relation between second sensor signals and second actions. In specific embodiments, the second device may be selected from the group consisting of a skin analysis device and a skin treatment device.

The term "kit of parts" especially refers to two or more items (parts) that may be distinct, but which are - in the context of the invention - designed to be used together. Especially, to two or more items (parts) may be designed to be arranged into a single arrangement, such as a device, apparatus or system. The term "kit of parts" may refer to the two or more items in a physical kit, but this is not necessarily the case. Together, the two or more items may provide a desired effect. The desired effect, herein, is especially that the first device prepares (in a first action) the (second) action of the second device or the second device is able to execute the action based on the first marking (and second marking) and the information provided by this second marking.

Hence, in embodiments the second device may be configured to execute a treatment. Therefore, the second device may be a skin treatment device. The skin treatment device may in embodiments comprises a functional unit selected from an UV radiator, a visible light radiator, a radio frequency (RF) generator, an ultrasound generator, a suction device, a needle actuator device, a cold plasma generator, an IR radiator, a massaging device, a scrubbing device, an exfoliation device, and an applicator device for applying an active ingredient comprising material.

In embodiments, the second action may comprise one or more of applying UV radiation to the skin part, applying visible light to the skin part, applying radio frequency (RF) waves to the skin part, applying ultrasound to the skin part, applying suction to the skin part, applying a needle to the skin part, including optionally penetrating into the skin of the skin part, applying a cold plasma to the skin part, applying IR radiation to the skin part, massaging the skin part, scrubbing the skin part, exfoliating the skin part, and applying an active ingredient comprising material to the skin part.

In specific embodiments, the active ingredient comprising material comprises one or more of a cosmetic material, a skin care material, and a pharmaceutical material. The active ingredient may e.g. include charcoal. Good results may be obtained with charcoal from the Poales order of plants, such as Poaceae. The Poales are an order of flowering plants in the monocotyledons, and includes families of plants such as the grasses (Poaceae), bromeliads, and sedges.

The second device may also comprise a distance holder. The distance holder(s) may be an edge, with the sensor configured within a cavity which may be at least partly be defined by the edge. When the second device is configured on the skin, the skin may close the cavity.

Note that in embodiments the first device may be configured to be used on the skin when executing the first action. In embodiments of the second device, however, it may not be necessary to execute the second action with the second device configured on the skin.

As indicated above, the second device may comprise a control system. This control system (or optionally an external (master) control system) may also include a memory. In embodiments, the memory may include the information of the predetermined relation between skin parameters and first markings. Amongst others, this control system may derive information on the condition, and on the basis thereon have the second device execute a treatment. As indicated above, in specific embodiments, the first marking may contain information about the conditions of the treatment to be executed, such as one or more of intensity of the treatment, the length in time of a treatment, the number of treatments (including optionally the associated time of each treatment), such as e.g. a treatment scheme. The second device may read this from the first marking and execute the corresponding treatment scheme. For instance, the device may switch of the application of the functional unit when the treatment time has been executed. As the control system of the second device or to which the second device may be functionally coupled, also include a memory, the control system may in embodiments also prevent action of the functional unit when the treatment scheme has fully been executed. This may prevent over treatment, such as e.g. an overdosis of UV radiation.

Hence, the invention also provides in an aspect a system comprising (i) the screening device as defined herein, and (ii) a second device (such as defined herein).

As indicated above, especially the second device may be selected from the group consisting of a skin analysis device and a skin treatment device. In embodiments, the second device may (thus) especially be configured to execute a second action selected from the group consisting of executing an analysis and executing a treatment, respectively, wherein the second action is selected out of a predetermined set of second actions, wherein the second device comprises a second sensor configured to generate a second sensor signal in response to one or more of (a) the first marking, (b) the background of the first marking, and (c) the first marking and its background. Examples of second actions are - amongst others - provided above.

For instance, a first marking in the form of a code may have been provided by providing the code (in e.g. black) itself, like a bar code, or providing a negative (in black) of the code (like a printed frame defining the bars at position where nothing has been printed), such that in embodiments the code is effectively the skin surrounded by the negative.

In embodiments, the second device further comprises a second control system configured to select the second action in response to the second sensor signal based on a predefined relation between second sensor signals and second actions. Especially, further one or more of the following may apply: (a) the first control system is configured to control the second control system, (b) the second control system is configured to control the first control system, and (c) the system comprises a master control system configured to control the first control system and the second control system. Hence, one of the control systems may be a master control system and the other control system(s) may be (a) slave control system(s).

The master control system may be comprised by a system comprising the first device and the second device. However, the master control system may also be cloud based.

In embodiments, the screening device may comprise a (wireless) communication facility. In embodiments, the second device may comprise a second (wireless) communication facility. In such embodiments, the master control system may communicate with the screening device and the second device. In this way, e.g. the master system may communicate the predetermined relation between skin parameters and first markings to the screening device and second device. It may also be possible in embodiments that the screening device sends the first sensor signal (or an adapted first sensor signal, such as enriched with information, like e.g. time) to the master control system, and the master control system controls the marking device (in dependence of the first sensor signal), in embodiments via the first control system of the screening device. It may also be possible in embodiments that the second device sends the second sensor signal (or an adapted second sensor signal) to the master control system, and the master control system controls the second device, more especially the second action thereof, especially in dependence of the second sensor signal.

In embodiments, the screening device may comprise a (wireless) communication facility and the second device may comprise a second (wireless) communication facility. In embodiments, one of the device communicates the predetermined relation between skin parameters and first markings to the other, such that the screening device knows which first markings have to be generated in dependence of the first sensor signal, or such that the second device know which second action has to be executed in dependence of the first marking (i.e. the skin parameter(s) that can be derived from the first marking. The term "(wireless) communication facility" and similar terms may e.g. refer to a system or device having a facility to communication, such as via Wi-Fi or Bluetooth or ZigBee, etc.

As can be concluded from the above, in specific embodiments, the first control system of the screening device may be a master control system, configured also to control the second control system of the second device. In alternative specific embodiments, the second control system of the second device may be a master control system, configured also to control the first control system of the screening device.

In yet a further aspect, the invention also provides a method for screening a skin part, comprising: (i) determining a skin parameter of the skin part, wherein the skin parameter is a skin condition parameter of the skin part, and wherein the skin parameter is selected out of a predetermined set of skin parameters; and (ii) generating to the skin part a first marking, selected out of a (predetermined) set of markings, according to a predetermined relation between skin parameters and markings.

Especially, the screening method may be executed with the screening device as described herein.

As indicated above, screening may be a first action, which may be followed by a second action. Hence, in specific embodiments the method may further comprise executing a second action selected from the group consisting of executing an analysis of the skin part and executing a treatment of the skin part, respectively, wherein the second action is executed in dependence of information derived from the first skin marking.

Especially, the second action may be executed with the second device as described herein.

Especially, the method is a non-medical method. Especially, the method is a cosmetical method.

In yet a further aspect, the invention also provides a computer program product, when running on a computer which is functionally coupled to or comprised by the screening device as defined herein or the system as defined herein, may be capable of bringing about the method as defined herein. Especially, hereby the respective screening device or second device is functionally configured to part of the skin to execute the screening or second action, respectively. The computer may be an external computer, comprising e.g. the master control system. Alternatively, the screening device comprises the computer, comprising e.g. the first control system, or the second device comprises the computer, comprising e.g. the second control system.

Also, in yet a further aspect the invention provides a data carrier having stored thereon program instructions, which when executed by the system as defined herein causes the system to execute the method as defined herein.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1b schematically depict some embodiments of the screening device;
Figs. 2a-2b very schematically depict some examples of skin parts as well as embodiments of the first marking;
Figs. 3a-3b very schematically depict some embodiments of a kit of parts as well as of a system, respectively.

The schematic drawings are not necessarily to scale.

### DETAILED DESCRIPTION OF THE EMBODIMENTS

In an embodiment, the invention provides a skin detection device or first device with integrated skin marking device to detect characteristic skin areas. The device may comprising a sensing element to detect skin areas of interest visible (e.g. pigmentation, pimples, wounds, scars, ...) and invisible (e.g. early pimples, low and high blood perfused areas, fragile or sensitive skin, skin elasticity, ...) for the user.

In embodiments, the device may comprise a sensor, which may comprise one or more a camera, a thermal imager, a force applicator, a topography evaluator, etc.. The sensor may generate a single image or a movie, which may be used to define a skin parameter.

Hence, the first device may be run with software (e.g. internal, cloud based, ...) and algorithms for data extraction of skin feature of interest may be applied.

Further, the first device may comprise a skin area addressing marker, which may e.g. place a UV, visible, or IR marker onto the skin (e.g. ink spray or application, fluorescent particles, plasmonic particles). The marker may include a circular shape (boundary), a cross (marker), etc. etc. In embodiments, optionally a coded marker corresponding to a specific type of features in the skin (e.g. using shape, bar code, color) or to an indication of the level/state of the skin condition may be applied.

With another device, a treatment device (or an analysis device), generally indicated as "second device", the indicated skin part may be manipulated, such as create local inflammation, alter blood absorption wavelength (methemoglobin), cool or warm locally the skin part, change surface properties (e.g. hydrophobic/hydrophilic, friction, optical coupling), etc.

A treatment device (embodiment of a second device) may comprise a detection element to detect skin area addressing marker. The action of second device may be based on detected skin area addressing marker. The marker may contain information where locally the treatment device should start the treatment and where the treatment device should stop the treatment. The marker may contain information about the treatment. Hence, the marker may induce an altering of the current treatment setting based on the marker. As will be clear to a person skilled in the art, different markers could have different treatment adjustments.

In the embodiments where the treatment device may provide manipulation of skin area, this may e.g. include create a local inflammation. This can be achieved by inducing heat (e.g. light pulse, RF pulse, Ultrasound pulse, ...) or by mechanical means (skin abrasion, indentation, ...) or by chemical means. Manipulation may also include altering blood absorption wavelength (methemoglobin). In particular, this can be obtained using high intensity light pulse. Manipulation may also include cooling or heating locally the skin part, e.g. using a Peltier element and/or a heater. Manipulation may also include (temporarily) changing surface properties (e.g. hydrophobic/hydrophilic, friction, or in embodiments optical coupling). Hydrophilicity may be changed chemically, e.g. application of a topical material. Friction may e.g. imply adding a lubricant, changing the skin condition (e.g. moisturizing), etc..

In embodiments, the detection device places material (chemicals) on the target area or around the target area to improve the treatment. In embodiments, the detection device may e.g. provide a layer to protect a part from the treatment the treatment device. In embodiments, the detection device (and/or treatment device) may provide material, such as particles to enhance a photo thermal treatment. In embodiments, the detection device (and/or treatment device) may provide a radiation coupling gel to improve light incoupling into the skin).

In yet other embodiments, the markers created on the skin can be used by another skin detection device for improving the diagnostics of a skin condition. E.g. a first device may be able to detect the location of an early pimple by using blood perfusion and place a marker. The second device may be used for finding out the exact state of the pimple using ultrasound imaging.

In yet other embodiments, the markers can be used by another detection device for registering the position of the marker and sub-lying skin condition. E.g. a camera can be used to detect the position of the marker and track its evolution over time. The marker will provide the information of the position on the face, the type of condition and the level/state of the condition, which could not be detected by a simple camera.

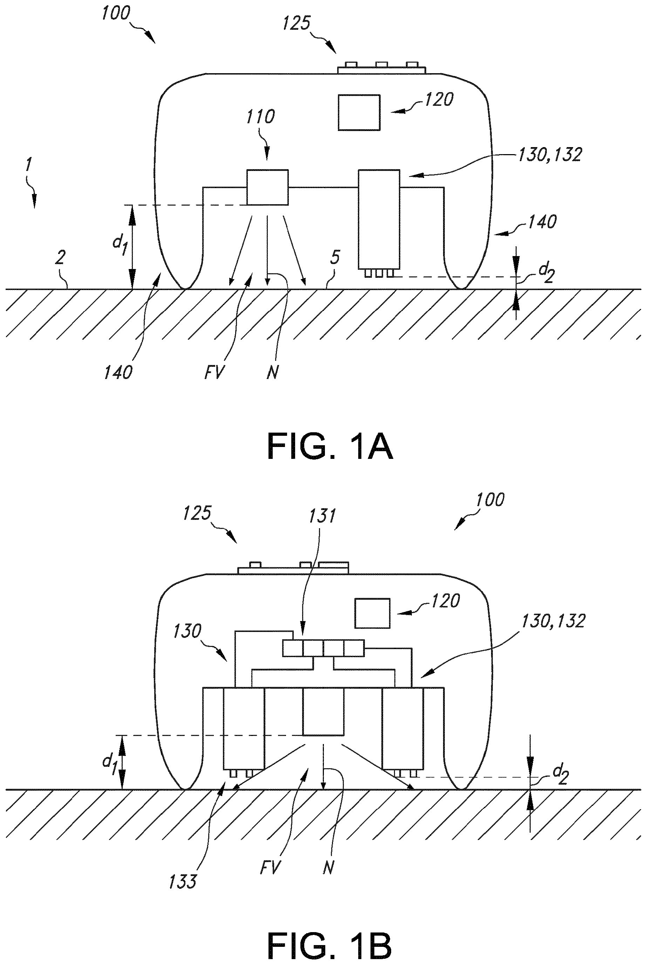

Figs. 1a-1b schematically depicts embodiments of a screening device 100 for screening a skin part, wherein the screening device 100 comprises a first sensor 110, a first control system 120, and a marking device 130. The first sensor 110, and first control system 120 are configured to determine a skin parameter of a skin part. The skin is indicated with reference 1. The outer layer or stratum corneum of the skin 1 is indicated with reference 2. The skin part is indicated with reference 5.

The first sensor 110 is configured at a predetermined distance d1 from the skin. The predetermined distance d1 is selected from the range of 0-50 mm. Here, by way of example the predetermined distance d1 is > 0 mm.

As indicated above, the skin parameter is a skin condition parameter of the skin part 5. The skin parameter is selected out of a predetermined set of skin parameters.

The marking device 130 is configured to generate to the skin part 5 a first marking, selected out of a (predetermined) set of first markings, according to a predetermined relation between skin parameters and first markings. The first marking contains information related to one or more of a depth of the condition, an extent over the skin of the condition, a severity of the condition, a stage of the condition, etc.. For instance, the marking device 130 comprises one or more of a printer 132 (see Fig. 1a) and a spray device 133 (see Fig. 1b).

The marking device 130 may be configured at a second distance d2 from the skin during operation of the screening device 100. Hence, when the screening device 100 is position on the skin 1, the sensor device may be configured at distance d1 and the marking device 130 may be configured at distance d2 from the skin 1, especially due to the distance holders 140. Here, by way of example the predetermined distance d2 is > 0 mm.

In specific embodiments, this distance may change with time with a distance d2>0 mm when not generating the first marking to the skin, and with distance d2=0 during generating the first marking to the skin. Hence, in specific embodiments the marking device may also comprise a movable element. This may e.g. allow a movement (in a z-direction / perpendicular to the skin).

The distance holder(s) may be an edge, with the sensor and marking device configured within a cavity which may be at least partly be defined by the edge.

Note that the distance holder(s) 140 may define a cavity, which is closed by the skin 1, when the device 100 is configured on the skin.

In embodiments, the first sensor 110 may comprise one or more of a camera, a thermal imager, a topographical imager, an ultrasound sensor, a radiofrequency sensor, and a capacitance sensor, etc.

In Figs. 1a-1b, the first sensor 110 has a field of view FV and a nadir N. The marking device 130 may be configured to provide the first marking and/or the area marking (see below) (to the skin part 5) within the field of view FV. Alternatively, the marking device 130 may be configured to provide a plurality of the first marking and/or a plurality of the area markings (to the skin part 5) at an edge of the field of view FV or outside the field of view FV.

When at both sides of the field of view FV the marking device can provide a mark, the area between the markings may e.g. indicate the skin part 5 to be subjected to the second action (see also below).

Reference 125 refers to a user interface, such as a graphical user interface.

Reference(s) 140 refers to a distance holder / distance holders, which allow the first sensor 110, or at least part thereof, and/or the marking device 130, or at least part thereof, be configured at a predetermined distance from the skin. The predetermined distance for the first sensor is indicated with d1; the predetermined distance for the marking device 130 is indicated with d2.

Fig. 1b further schematically depicts an embodiment wherein the screening device 100 comprises a plurality of cartridges 131 configured to host pigments or to host luminescent materials, for providing the first marking(s).

The skin parameter is especially selected from local pigmentation excess, local pigmentation scarcity, local pigmentation anomaly, pimple formation, a pimple, a wound, a scar, a local low blood perfused area, a local high blood perfused area, a local blood perfusion anomaly, a fragile skin part, a sensitive skin part, wrinkles, skin hydration, skin oiliness, and a local skin elasticity anomaly.

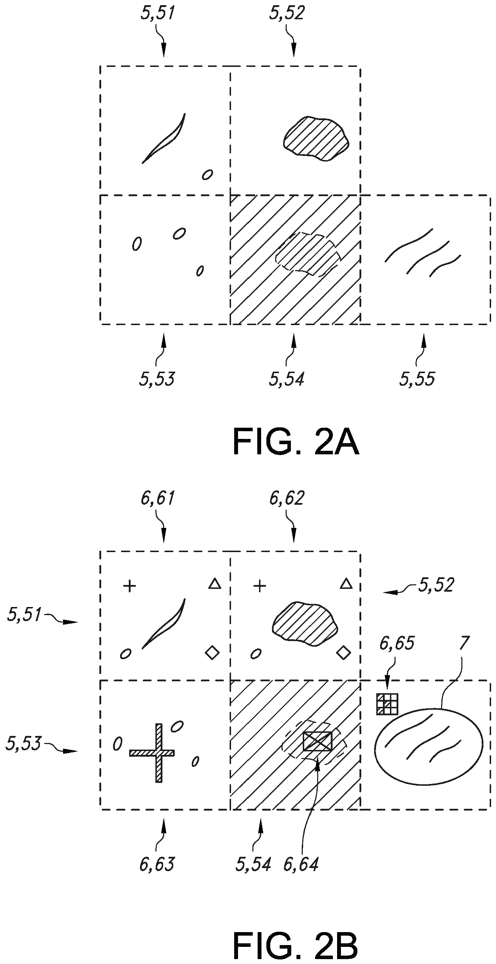

Figs. 2a and 2b very schematically depict five examples of skin parts 5. Reference 51 refers to a skin part 5 comprising a scar. Reference 52 refers to a skin part 5 having a pigment anomaly, which might e.g. be a risk for tumor formation. Reference 53 refers to a skin part 5 which has no anomaly or special condition at all, and can be considered normal and healthy. It may e.g. comprise a few sunspots, but these are only drawn by way of example. Reference 54 indicates a skin part with a subcutaneous inflammation. Reference 55 indicates a skin part with wrinkles.

The marking device is configured to generate to the skin part 5 a colored first marking, selected out of a (predetermined) set of colored first markings, according to a predetermined relation between skin parameters and the colored first markings. Alternatively or additionally, the marking device 130 is configured to generate to the skin part 5 a patterned first marking, selected out of a (predetermined) set of patterned first markings, according to a predetermined relation between skin parameters and the patterned first markings. For instance, the screening device 100 may comprise a printer 132 (see Fig. 1a), wherein the printer 132 is configured to print patterned first markings, according to a predetermined relation between skin parameters and the patterned first markings.

Fig. 2b schematically depicts a plurality of possible markings 6. However, these are only examples of (first) markings. First marking 61 may in this configuration show the type of skin, i.e. comprising a scar, like first marking 62 this may do for the skin part 5,52 having a pigment anomaly. Both first markings 61 and 62 also provide information of the skin part to be treated, as the first marking(s) surround the relevant skin part 5 (or part of the skin part 5). First markings 63 and 64 may also provide information about the respective condition. Further, these markings may e.g. also contain information about the area to be treated around the first marking 63 and 64, such as e.g. a circle or square around a center of gravity of the first markings 63 or 64, respectively. First marking 65 may schematically depict a QR code or other coded marking 6. The marking device may also be configured to generate to the skin part 5 an area marking for indicating the area of the skin part 5 associated to the (defined) skin parameter. Reference 7 schematically depicts such area marking, which may be a pigment containing material or dye containing material surrounding the skin part 5 or part of the skin part 5 to be subjected to the second action.

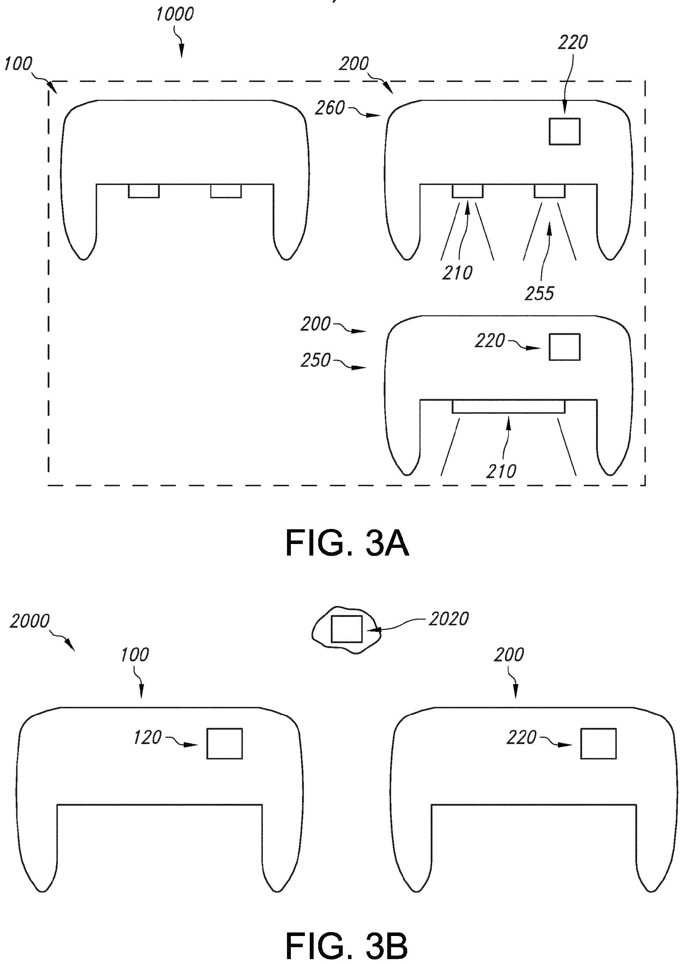

Fig. 3a schematically depicts an embodiment of a kit 1000 of parts comprising the screening device 100 and a second device 200. Here, by way of example two second devices 200 are schematically depicted. The second device 200 may be selected from the group consisting of a skin analysis device 250 and a skin treatment device 260.

Hence, the second device(s) 200 is (are) configured to execute a second action selected from the group consisting of executing an analysis and executing a treatment, wherein the second action is selected out of a predetermined set of second actions.

The second device 200 comprises a second sensor 210 configured to generate a second sensor signal in response to one or more of a the first marking, the background of the first marking, and the first marking and its background, wherein the second device 200 further comprises a second control system 220 configured to select the second action in response to the second sensor signal based on a predefined relation between second sensor signals and second actions. The schematic version of the second device 250 configured to execute an analysis, i.e. second device 250 by way of example comprises a sensor which can both recognize the first marking and execute the analysis. Of course, for these functions also different sensors may be applied.

The skin treatment device 260 may comprise a functional unit 255 selected from an UV radiator, a visible light radiator, a radio frequency RF generator, an ultrasound generator, a suction device, a needle actuator device, a cold plasma generator, an IR radiator, a massaging device, a scrubbing device, an exfoliation device, and an applicator device for applying an active ingredient comprising material, etc.

Fig. 3b very schematically depicts a system 2000 comprising the screening device 100 and the second device 200. One or more of the following may apply: (a) the first control system 120 is configured to control the second control system 220, (b) the second control system 220 is configured to control the first control system 120, and (c) the system 2000 comprises a master control system 2020 configured to control the first control system 120 and the second control system 220. Here, by way of example the latter variant is schematically depicted, which, by way of example the master control system 2020 being configured in the cloud.

In alternative embodiments of the kit 1000 and/or in alternative embodiments of the system 1000, the first control system 120 or the second control system 220 comprises the master control system.

Further, in embodiments the first device and or the second device may comprise communication facilities, respectively, such as based on Bluetooth or Wi-Fi, e.g. to communicate the predetermined relation, or the skin parameter, etc.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" includes also embodiments wherein the term "comprises" means "consists of'.

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. A screening device (100) for screening a skin part, wherein the screening device (100) comprises a first sensor (110), a first control system (120), and a marking device (130), wherein:
- the first sensor (110) and first control system (120) are configured to determine a skin parameter of a skin part when the first sensor (110) is configured at a predetermined distance (d1) from the skin, wherein the skin parameter is a skin condition parameter of the skin part, and wherein the skin parameter is selected out of a predetermined set of skin parameters; and
- the marking device (130) is configured to generate to the skin part a first marking, selected out of a set of first markings, according to a predetermined relation between skin parameters and first markings.

2. The screening device (100) according to claim 1, wherein the first sensor (110) comprises one or more of a camera, a thermal imager, a topographical imager, an ultrasound sensor, a radiofrequency sensor, and a capacitance sensor, wherein the first marking contains information related to one or more of a depth of the condition, an extent over the skin of the condition, a severity of the condition, and a stage of the condition, and wherein the predetermined distance (d1) is selected from the range of 0-50 mm.

3. The screening device (100) according to any one of the preceding claims, wherein the skin parameter is selected from local pigmentation excess, local pigmentation scarcity, local pigmentation anomaly, pimple formation, a pimple, a wound, a scar, a local low blood perfused area, a local high blood perfused area, a local blood perfusion anomaly, a fragile skin part, a sensitive skin part, wrinkles, skin hydration, skin oiliness, and a local skin elasticity anomaly.

4. The screening device (100) according to any one of the preceding claims, wherein the marking device (130) is configured to generate to the skin part a colored first marking, selected out of a set of colored first markings, according to a predetermined relation between skin parameters and the colored first markings.

5. The screening device (100) according to any one of the preceding claims, wherein the marking device (130) is configured to generate to the skin part a patterned first marking, selected out of a set of patterned first markings, according to a predetermined relation between skin parameters and the patterned first markings.

6. The screening device (100) according to claim 5, wherein the screening device (100) comprises a printer (132), wherein the printer (132) is configured to print patterned first markings, according to a predetermined relation between skin parameters and the patterned first markings.

7. The screening device (100) according to any one of the preceding claims, wherein the marking device (130) is configured to generate to the skin part an area marking for indicating the area of the skin part associated to the skin parameter.

8. The screening device (100) according to any one of the preceding claims, wherein the first sensor (110) has a field of view (FV), wherein the marking device (130) is configured to provide to the skin part the first marking and/or the area marking according to claim 7 within the field of view (FV), or wherein the marking device (130) is configured to provide at the predetermined distance (d1) a plurality of the first marking and/or a plurality of the area markings according to claim 7 at an edge of the field of view (FV) or outside the field of view (FV).

9. The screening device (100) according to any one of the preceding claims, wherein the marking device (130) comprises one or more of a printer (132) and a spray device (133).

10. A kit (1000) of parts comprising (i) the screening device (100) according to any one of the preceding claims 1-9, and (ii) a second device (200), wherein the second device (200) is configured to execute a second action selected from the group consisting of executing an analysis and executing a treatment, wherein the second action is selected out of a predetermined set of second actions, wherein the second device (200) comprises a second sensor (210) configured to generate a second sensor signal in response to one or more of (a) the first marking, (b) the background of the first marking, and (c) the first marking and its background, wherein the second device (200) further comprises a second control system (220) configured to select the second action in response to the second sensor signal based on a predefined relation between second sensor signals and second actions.

11. The kit (1000) of parts according to claim 10, wherein the second device (200) is configured to execute a treatment, wherein the skin treatment device (260) comprises a functional unit (255) selected from an UV radiator, a visible light radiator, a radio frequency (RF) generator, an ultrasound generator, a suction device, a needle actuator device, a cold plasma generator, an IR radiator, a massaging device, a scrubbing device, an exfoliation device, and an applicator device for applying an active ingredient comprising material.

12. A system (2000) comprising (i) the screening device (100) according to any one of the preceding claims 1-9, and (ii) a second device (200), wherein the second device (200) is selected from the group consisting of a skin analysis device (250) and a skin treatment device (260), wherein the second device (200) is configured to execute a second action selected from the group consisting of executing an analysis and executing a treatment, respectively, wherein the second action is selected out of a predetermined set of second actions, wherein the second device (200) comprises a second sensor (210) configured to generate a second sensor signal in response to one or more of (a) the first marking, (b) the background of the first marking, and (c) the first marking and its background, wherein the second device (200) further comprises a second control system (220) configured to select the second action in response to the second sensor signal based on a predefined relation between second sensor signals and second actions, wherein further one or more of the following applies: (a) the first control system (120) is configured to control the second control system (220), (b) the second control system (220) is configured to control the first control system (120), and (c) the system (2000) comprises a master control system (2020) configured to control the first control system (120) and the second control system (220).

13. A method for screening a skin part, comprising:
- determining a skin parameter of the skin part, wherein the skin parameter is a skin condition parameter of the skin part, and wherein the skin parameter is selected out of a predetermined set of skin parameters; and
- generating to the skin part a first marking, selected out of a set of markings, according to a predetermined relation between skin parameters and markings.

14. The method according to claim 13, further comprising executing a second action selected from the group consisting of executing an analysis of the skin part and executing a treatment of the skin part, respectively, wherein the second action is executed in dependence of information derived from the first skin marking.

15. A computer program product, when running on a computer (310) which is functionally coupled to or comprised by the screening device (100) according to any one of the preceding claims 1-9 or the system (2000) according to claim 12, is capable of bringing about the method according to any one of the preceding claims 13-14.
